# EUROPEAN PATENT SPECIFICATION

(11) **EP 3 964 578 B1**
(45) Date of publication and mention of the grant of the patent: **13.05.2026**
(21) Application number: 20798283.6
(22) Date of filing: 29.04.2020
(51) Int. Cl.: C12Q 1/6886

(54) **METHYLATION-BASED MODIFIED TUMOR MARKER STAMP-EP8 AND APPLICATION THEREOF**
METHYLIERUNGSBASIERTER MODIFIZIERTER TUMORMARKER STAMP-EP8 UND ANWENDUNG DAVON
MARQUEUR TUMORAL STAMP-EP8 BASÉ SUR UNE MODIFICATION DE MÉTHYLATION ET SON APPLICATION

(30) Priority: 30.04.2019 CN 201910363470
(43) Date of publication of application: 09.03.2022
(73) Proprietor: Shanghai Epiprobe Biotechnology Co., Ltd., Shanghai 200233 (CN)
(72) Inventor: LI, Zhenyan, Shanghai 200233 (CN); DONG, Shihua, Shanghai 200233 (CN)
(74) Representative: Kirkpatrick
(86) International application number: PCT/CN2020/087885
(87) International publication number: WO 2020/221315

(56) References cited:
- WO-A1-2016/083360
- WO-A1-2016/207656
- WO-A1-2017/072292
- WO-A1-2017/072292
- WO-A2-2017/143296
- CN-A- 105 886 657
- CN-A- 109 652 541
- CN-A- 109 971 860
- DATABASE NUCLEOTIDE 2 August 2020 (2020-08-02), ANONYMOUS: "Homo sapiens NK2 homeobox 6 (NKX2-6), RefSeqGene on chromosome 8", XP055868352, retrieved from GENBANK Database accession no. NG_030636.
- BREEZY M LINDQVIST ET AL.: "Whole Genome DNA Methylation Signature of HER2-positive Breast Cancer", EPIGENETICS, vol. 9, no. 8, 8 July 2014 (2014-07-08), XP055200041, ISSN: 1559-2294, DOI: 20200529135646Y

## Description

### Technical field

The disclosure is in the field of markers for diagnosing diseases. More specifically, the disclosure relates to a methylation based tumor marker STAMP, Specific Tumor Aligned Methylation of Pan-cancer, and use thereof.

### BACKGROUND OF DISCLOSURE

Tumors have been considered a genetic disease for decades. Several large-scale systematic sequencings for human have confirmed that the number of somatic mutations in cancer tissues is significantly less than expected. These results suggest that cancer is not a simple genetic disease.

In order to diagnose tumor, many new tumor markers have been discovered and used for clinical diagnosis in recent years. Before 1980, tumor markers were mainly hormones, enzymes, proteins and other cell secretions, such as carcinoembryonic antigen (CEA) and alpha fetoprotein (AFP) used as markers of gastric cancer, liver cancer and other tumors, carbohydrate antigen 125 (CA125) used as a marker of cervical cancer, and prostate specific antigen (PSA) used as a marker of prostate cancer. Although these tumor markers are still used in clinic, their sensitivity and accuracy have been difficult to meet the clinical needs.

More and more evidences show that small changes in epigenetic regulation play an important role in tumors. Epigenetics is a subject that studies that the heritable change of gene function without a change of DNA sequence, which eventually leads to the change of phenotype. Epigenetics mainly includes DNA methylation, histone modification, changes of microRNA level and other biochemical processes. DNA methylation is one of the epigenetic mechanisms, refers to the process of transferring methyl from S-adenosylmethionine (methyl donor) to specific bases under the catalysis of DNA methyltransferase. However, DNA methylation in mammals mainly occurs at the C of 5'-CpG-3', which results in 5-methylcytosine (5mC).

Fluid biopsy is a technique for the diagnosis and prediction of tumors using circulating tumor cells or circulating tumor DNA as detection targets. The technology has many shortcomings. First, the sensitivity and specificity are not good enough. The tumor itself is heterogeneous, including a variety of subtypes of cell populations. The proportion of tumor DNA in clinical samples, especially blood samples, is very low. The existing tumor markers are difficult to meet the sensitivity of clinical requirements, and it is easy to cause misdiagnosis. Second, one marker has good effect only for one or a few kinds of tumors. As the DNA sources in blood are very complex, the existing tumor markers cannot solve the complex problems of tumor source and metastasis. Because of these complexities, it is difficult for many DNA methylation tumor markers to have a unified standard in clinical application, which seriously affects the sensitivity and accuracy of the markers.

In the previous study of the inventor, some tumor markers based on methylation modification were found. WO 2016/083360, WO 2016/207656, WO 2017/072292 and WO 2017/143296 disclose the use of NKX2-6/chromosome 8 sequences for the detection of lung, bladder, breast or prostate cancer, respectively. However, to provide more ways for tumor diagnosis, it is still necessary to find more new tumor markers.

### SUMMARY OF DISCLOSURE

The object of the disclosure is to provide a method for detecting tumor based on abnormal hypermethylation of specific sites in tumor using DNA methylation modification as a tumor marker.

The first aspect of the present disclosure provides the method according to claim 1. Disclosed, but not claimed, is an isolated first polynucleotide, including: (a) a polynucleotide with a nucleotide sequence as shown in SEQ ID NO: 1; (b) a fragment of the polynucleotide of (a), having at least one (such as 2-191, more specifically 3, 5, 10, 15, 20, 25, 30, 40, 50, 60, 70, 90, 110, 130, 150, 170, 180, 190) modified CpG site; and/or (c) a nucleic acid (such as the polynucleotide with a nucleotide sequence as shown in SEQ ID No: 3) complementary to the polynucleotide or fragment of (a) or (b). Disclosed, but not claimed, modifications include 5-hydroxymethylation(5hmC), 5-formylcytosine(5fC) or 5-carboxylcytosine(5-caC).

In a preferable embodiment, the fragment of the polynucleotide contains bases at: positions 204 to 223 (including methylation sites 021 to 024). Disclosed, but not claimed, fragments include: positions 1 to 478 (including methylation sites 001 to 040) of SEQ ID NO: 1 or 2; positions 513 to 1040 (including methylation sites 041 to 077) of SEQ ID NO: 1 or 2; positions 1082 to 1602 (including methylation sites 078 to 114) of SEQ ID NO: 1 or 2; positions 1621 to 2117 (including methylation sites 115 to 153) of SEQ ID NO: 1 or 2; positions 2160 to 2700 (including methylation sites 154 to 191) of SEQ ID NO: 1 or 2.

Disclosed, but not claimed, is an isolated polynucleotide, which is converted from the first polynucleotide, and as compared with the sequence of the first polynucleotide, its cytosine C of the CpG site(s) with modification is unchanged, and the unmodified cytosine is converted into T or U. It may be converted from the polynucleotide corresponding to the first polynucleotide by bisulfite treatment. The polynucleotide may include: (d) a polynucleotide with a nucleotide sequence as shown in SEQ ID NO: 2 or 4; (b) a fragment of the polynucleotide of (d), having at least one (such as 2-191, more specifically 3, 5, 10, 15, 20, 25, 30, 40, 50, 60, 70, 90, 110, 130, 150, 170, 180, 190) CpG site with modification. The fragment of the polynucleotide may contain bases at: positions 204 to 223 (including methylation sites 021 to 024); positions 1 to 478 (including methylation sites 001 to 040) of SEQ ID NO: 1 or 2; positions 513 to 1040 (including methylation sites 041 to 077) of SEQ ID NO: 1 or 2; positions 1082 to 1602 (including methylation sites 078 to 114) of SEQ ID NO: 1 or 2; positions 1621 to 2117 (including methylation sites 115 to 153) of SEQ ID NO: 1 or 2; positions 2160 to 2700 (including methylation sites 154 to 191) of SEQ ID NO: 1 or 2; positions 2478 to 2497 (corresponding to methylation sites 021 to 024); positions 2223 to 2700 (corresponding to methylation sites 001 to 040) of SEQ ID NO: 4 or 3; positions 1661 to 2188 (corresponding to methylation sites 041 to 077) of SEQ ID NO: 4 or 3; positions 1099 to 1619 (corresponding to methylation sites 078 to 114) of SEQ ID NO: 4 or 3; positions 584 to 1080 (corresponding to methylation sites 115 to 153) of SEQ ID NO: 4 or 3; positions 1 to 541 (corresponding to methylation sites 154 to 191) of SEQ ID NO: 4 or 3.

A further aspect of the disclosure provides a use of the polynucleotide described herein in manufacture of a tumor detection kit according to claim 12.

In a preferable embodiment, the tumors include (but are not limited to): hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer (cancer of the esophagus), gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

In another preferable embodiment, samples of the tumor include but are not limited to: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

A further aspect of the disclosure provides a method of preparing a tumor detection agent according to claim 9. Disclosed but not claimed is a method of preparing a tumor detection agent including: providing the polynucleotide described in the first or second aspect, designing a detection agent for specifically detecting the modification on CPG site(s) of a target sequence which is the full length or fragment of the polynucleotide; wherein, the target sequence has at least one (such as 2-191, more specifically, 3, 5, 10, 15, 20, 25, 30, 40, 50, 60, 70, 90, 110, 130, 150, 170, 180, 190) modified CpG site; preferably, the detection agent includes (but is not limited to) primers or probes.

A further aspect of the disclosure provides a combination of agents which specifically detect the modification on CPG site(s) of a target sequence according to claim 10. Disclosed but not claimed is an agent or a combination of agents which specifically detect the modification on CPG site(s) of a target sequence, which is the full length or fragment of any of the polynucleotides described herein and has at least one (such as 2-191, more specifically, 3, 5, 10, 15, 20, 25, 30, 40, 50, 60, 70, 90, 110, 130, 150, 170, 180, 190) modified CpG site.

The agent or combination of agents may be for a gene sequence containing the target sequence (designed based on the gene sequence), and the gene sequence includes gene Panels or gene groups.

The detection agent may comprise: primers or probes.

Disclosed, but not claimed, are: the primers shown in SEQ ID NO: 3 and 4; the primers shown in SEQ ID NO: 7 and 8; the primers shown in SEQ ID NO: 9 and 10; the primers shown in SEQ ID NO: 11 and 12; the primers shown in SEQ ID NO: 13 and 14.

In a further aspect of the disclosure, a use of the agent or combination of agents described herein in the manufacture of a kit for detecting tumors according to claim 11 is provided; preferably, the tumors include (but are not limited to): hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

The seventh aspect of the present disclosure provides a detection kit according to claim 14; preferably, each agent is located in an independent container.

In a preferable embodiment, the kit also includes: bisulfite, DNA purification agent, DNA extraction agent, PCR amplification agent and/or instruction for use (indicating operation steps of the detection and a result judgment standard).

In an embodiment of the disclosure, a method for detecting the methylation profile of a sample *in vitro* is provided, including: (i) providing the sample and extracting the nucleic acid; (ii) detecting the modification on CPG site(s) of a target sequence in the nucleic acid of (i), wherein the target sequence is the polynucleotide described herein or the polynucleotide converted therefrom as described herein.

In step (3), the analysis methods may include pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR (MSP), or their combination, as well as *in vitro* detection and *in vivo* tracer detection for the combined gene group of partial or all of the methylation sites in the sequence shown in SEQ ID NO: 1. In addition, other methylation detection methods and newly developed methylation detection methods in the future can be applied to the disclosure.

Step (ii) may include: (1) treating the product of (i) to convert the unmodified cytosine into uracil; preferably, the modification includes 5-methylation, 5-hydroxymethylation, 5-formylcytosine or 5-carboxylcytosine; preferably, treating the nucleic acid of step (i) with bisulfite; and (2) analyzing the modification of the target sequence in the nucleic acid treated by (1).

The abnormal methylation profile may be the high level of methylation of C in CPG(s) of the polynucleotide.

The methylation profile detecting method may not be for the purpose of directly obtaining the diagnosis result of a disease, or may not be a diagnostic method.

A further disclosed, but not claimed, aspect of the disclosure provides a tumor diagnosis kit, including primer pair(s) designed based on the sequence described in the first or second aspect of the disclosure, and gene Panels or gene group containing the sequence, to obtain the characteristics of normal cells and tumor cells through DNA methylation detection.

### BRIEF DESCRIPTION OF DRAWINGS

Figure 1, BSP assay for methylation levels of the methylation sites 001 to 040 in SEQ ID NO:1 region in lung cancer cells and normal lung cells. Dark squares indicate "methylated".
Figure 2, BSP assay for methylation levels of the methylation sites 041 to 077 in SEQ ID NO:1 region in lung cancer cells and normal lung cells. Dark squares indicate "methylated".
Figure 3, BSP assay for methylation levels of the methylation sites 078 to 114 in SEQ ID NO:1 region in lung cancer cells and normal lung cells. Dark squares indicate "methylated".
Figure 4, BSP assay for methylation levels of the methylation sites 115 to 153 in SEQ ID NO:1 region in lung cancer cells and normal lung cells. Dark squares indicate "methylated".
Figure 5, BSP assay for methylation levels of the methylation sites 154 to 191 in SEQ ID NO:1 region in lung cancer cells and normal lung cells. Dark squares indicate "methylated".
Figure 6, comparison of methylation values of STAMP-EP8 between non-cancer tissues and cancer tissues for clinical samples of leukemia.
Figure 7, comparison of methylation values of STAMP-EP8 between paracancerous samples and cancer tissue samples for clinical samples of breast cancer.
Figure 8, comparison of methylation values of STAMP-EP8 between paracancerous samples and cancer tissue samples for clinical samples of colorectal cancer.
Figure 9, comparison of methylation values of STAMP-EP8 between paracancerous samples and cancer tissue samples for clinical samples of esophageal cancer.
Figure 10, comparison of methylation values of STAMP-EP8 between paracancerous samples and cancer tissue samples for clinical samples of liver cancer.
Figure 11, comparison of methylation values of STAMP-EP8 between paracancerous samples and cancer tissue samples for clinical samples of lung cancer.
Figure 12, comparison of methylation values of STAMP-EP8 between pancreatic samples and cancer tissue samples for clinical samples of pancreas cancer.

### DETAILED DESCRIPTION

The inventor is committed to the research of tumor markers. After extensive research and screening, the inventor provides a universal DNA methylation tumor marker, STAMP (Specific Tumor Aligned Methylation of Pan-cancer). In normal tissues, STAMP was hypomethylated, while in tumor tissues, it was hypermethylated. It can be used for clinical tumor detection and as the basis of designing tumor diagnostic agents.

### Terms

As used herein, "isolated" refers to a material separated from its original environment (if the material is a natural material, the original environment is the natural environment). For example, in living cells, polynucleotides and polypeptides in their natural state are not isolated or purified, but the same polynucleotides or polypeptides will be isolated ones if they are separated from other substances existed in the natural state.

As used herein, "sample" includes substances suitable for DNA methylation detection obtained from any individual or isolated tissue, cell or body fluid (such as plasma). For example, the samples include but are not limited to: tissue samples, paraffin embedded samples, blood samples, pleural effusion samples, and alveolar lavage fluid samples, ascites and lavage fluid samples, bile samples, stool samples, urine samples, saliva samples, cerebrospinal fluid samples, cell smear samples, cervical scraping or brushing samples, tissue and cell biopsy samples.

As used herein, "hypermethylation" refers to high level of methylation, hydroxymethylation, formylcytosine or carboxylcytosine of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

### Gene marker

In order to find a useful target for tumor diagnosis, the inventor has identified the target of STAMP-EP8 after extensive and in-depth research. The methylation status of the sequence of STAMP-EP8 gene is significantly different between tumor tissues and non-tumor tissues. If the abnormal hypermethylation with a nucleotide sequence as of STAMP-EP8 gene of a subject is detected, the subject can be identified as having a high-risk of tumor. Moreover, the significant difference of STAMP-EP8 between tumor and non-tumor tissues exists in different kinds of tumors, including solid tumors and non-solid tumors.

The disclosure describes, but does not claim, an isolated polynucleotide, comprising the nucleotide sequence shown in the sequence of SEQ ID NO: 1 or SEQ ID NO: 3 (the reverse complementary sequence of SEQ ID NO: 1). For tumor cells, the polynucleotide contains 5-methylcytosine (5mC) at C positions of many 5'-CpG-3'. The disclosure also describes, but does not claim, a fragment of the polynucleotide with a nucleotide sequence as shown in SEQ ID NO: 1 or 3, having at least one (such as 2-191, more specifically 3, 5, 10, 15, 20, 25, 30, 40, 50, 60, 70, 90, 110, 130, 150, 170, 180, 190) methylated CpG site. The above polynucleotides or fragments can also be used in the design of detection agents or detection kits.

Described, but not claimed, is a fragment containing the residues 281-309 of SEQ ID NO: 1 (containing CpG sites 017-020). Based on the information provided by the present disclosure, other fragments can be selected.

In addition, gene Panels or gene groups containing the sequence shown in the SEQ ID NO: 1 or SEQ ID NO: 2 or a fragment thereof are described, but not claimed. For the gene Panel or gene group, the characteristics of normal cells and tumor cells can also be identified through DNA methylation detection.

The above polynucleotides can be used as the key regions for analysis of the methylation status in the genome. Their methylation status can be analyzed by various technologies known in the art. Any technique that can be used to analyze the methylation state can be applied to the present disclosure.

When treated with bisulfite, un-methylated cytosine(s) of the above polynucleotides will be converted into uracil, while methylated cytosine(s) remained unchanged.

The disclosure also describes, but does not claim, the polynucleotides obtained from the above polynucleotides (including the complementary chain (antisense chain)) after being treated with bisulfite, including the polynucleotides with a nucleotide sequence as shown in SEQ ID NO: 2 or SEQ ID NO: 4. These polynucleotides can also be used in the design of detection agents or detection kits.

The disclosure also describes, but does not claim, a fragment of the polynucleotides obtained from the above polynucleotides or the antisense strand thereof after being treated with bisulfite, wherein the fragment contains at least one (such as 2-191, more specifically 3, 5, 10, 15, 20, 25, 30, 40, 50, 60, 70, 90, 110, 130, 150, 170, 180, 190) methylated CpG site. The No. of each CpG site in the antisense strand corresponding to the number of the sense strand is readily obtained according to the content described by the present disclosure.

### Detection agents and kits

Based on the new discovery of the disclosure, a detection agent designed based on said polynucleotide(s) is also provided for detecting the methylation profile of polynucleotide(s) in the sample *in vitro.* The detection methods and agents known in the art for determining the sequence, variation and methylation of the genome can be applied in the disclosure.

Therefore, the disclosure provides a method of preparing a tumor detection agent according to claim 9, including: providing the polynucleotide, designing a detection agent for specifically detecting a target sequence which is the full length or fragment of the polynucleotide; wherein, the target sequence has at least one methylated CpG site.

The detection agent herein includes but is not limited to: primers or probes, etc.

For example, the agent is primer pair(s). Based on the sequence of the polynucleotide, those skilled in the art know how to design primer(s). The two primers are on each side of the specific sequence of the target gene to be amplified (including CpG sequence, for the gene region originally methylated, the primer is complementary with CpG, and for the gene region originally un-methylated, the primer is complementary with TpG). It should be understood that based on the new discovery of the disclosure, those skilled in the art can design a variety of primers or probes or other types of detection agents for CpG sites at different positions on the target sequence or their combinations. These primers or probes or other types of detection agents should be included in the technical solution of the disclosure. In a preferable example of the disclosure, the primers are: the primers shown in SEQ ID NO: 5 and 6, which could be used to amplify a product containing CpG sites 001-040 of SEQ ID NO: 1; or, the primers shown in SEQ ID NO: 15 and 16, which could be used to amplify a product containing CpG sites 021-024 of SEQ ID NO: 1.Disclosed, but not claimed are the primers shown in SEQ ID NO: 7 and 8, which could be used to amplify a product containing CpG sites 041-077 of SEQ ID NO: 1; the primers shown in SEQ ID NO: 9 and 10, which could be used to amplify a product containing CpG sites 078-114 of SEQ ID NO: 1; the primers shown in SEQ ID NO: 11 and 12, which could be used to amplify a product containing CpG sites 115-153 of SEQ ID NO: 1; the primers shown in SEQ ID NO: 13 and 14, which could be used to amplify a product containing CpG sites 154-191 of SEQ ID NO: 1.

The agent can also be a combination of agents (primer combination), including more than one set of primers, so that the multiple polynucleotides can be amplified respectively.

The disclosure also provides a kit according to claim 14 for detecting the methylation profile of polynucleotide in a sample *in vitro,* which comprises container(s) and the above primer pair(s) in the container(s).

In addition, the kit can also include various reagents required for DNA extraction, DNA purification, PCR amplification, etc.

In addition, the kit can also include an instruction for use, which indicates operation steps of the detection and a result judgment standard, for the application of those skilled in the art.

### Detection method

The methylation profile of a polynucleotide can be determined by any technique in the art (such as methylation specific PCR (MSP) or real-time quantitative methylation specific PCR, Methylight), or other techniques that are still developing and will be developed.

Quantitative methylation specific PCR (QMSP) can also be used to detect methylation level. It is a continuous optical monitoring method based on fluorescent PCR, which is more sensitive than MSP. It has high throughput and avoids electrophoresis based result analysis.

Other available technologies include conventional methods in the art such as pyrosequencing, bisulfite converstion sequencing, qPCR, second generation sequencing, whole genome methylation sequencing, DNA enrichment detection, simplified bisulfite sequencing or HPLC, and combined gene group detection. It should be understood that, on the basis of the new disclosure herein, these well-known technologies and some technologies to be developed in the art can be applied to the present disclosure.

A method of detecting the methylation profile of a polynucleotide in a sample *in vitro* is also described, but not claimed. The method is based on the follow principle: the un-methylated cytosine can be converted into uracil by bisulfite, which can be transformed into thymine in the subsequent PCR amplification process, while the methylated cytosine remains unchanged; therefore, after the polynucleotide is treated by bisulfite, the methylated site presents a polynucleotide polymorphism (SNP) similar to C/T. Based on the above principle, methylated and un-methylated cytosine can be distinguished by identifying the methylation profile of a polynucleotide in the sample.

The method includes: (a) providing samples and extracting genomic DNA; (b) treating the genomic DNA of step (a) with bisulfite, so as to convert the un-methylated cytosine in the genomic DNA into uracil; (c) analyzing whether the genomic DNA treated in step (b) contains an abnormal methylation profile.

The method can be used for: (i) analyzing whether a subject has tumor by detecting the sample of the subject; (ii) identifying a population having high-risk of tumor. The method needs not to be aimed at obtaining direct diagnosis results.

DNA methylation may be detected by PCR amplification and pyrosequencing. It should be understood by those in the art that DNA methylation detection is not limited to these methods, and any other DNA methylation detection method can be used. The primers used in PCR amplification are not limited to those provided in Examples.

Because of bisulfite treatment, in which un-methylated cytosine in genomic DNA are converted into uracil and then transformed into thymine in the subsequent PCR process, the sequence complexity of the genome will be reduced, and it will be more difficult to amplify specific target fragments by PCR. Therefore, in order to improve amplification efficiency and specificity, nested PCR may be preferable, wherein two sets of primers (outer primers and inner primers) are used in two successive runs of PCR, and the product from the first run undergoes a second run with the second set of primers. However, it should be understood that the detection methods suitable for the present disclosure are not limited thereto.

After the research and verification on clinical samples, the method of the disclosure provides very high accuracy in the clinical diagnosis of tumors. The disclosure can be applied to the fields of tumor auxiliary diagnosis, efficacy evaluation, prognosis monitoring, etc., thus has a high clinical value.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Example 1. Nucleic acid sequence for STAMP-EP8 detection

Based on research and screening, a tumor marker STAMP-EP8 is provided and its sequence is as follows: SEQ ID NO: 1 (chr8:23562476-23565175, Human/hg19), in which the underlined bases are methylated CpG sites, and each number below the underline indicates the site number.

CGGATCGCTACCGCGGCCTTGGGCTCAGTTCCTGGAGCCGGCCTCGGGTCCGGCCTCGGGTC
1 2 3 4 5 6 7 8
CCTCCTCGGCGGCGTCCACTGCTGCCGCAGAAGAGACGCTCCCAGGATTTGGTTTCCAGTGG
9 10 11 12 13
GAGAAGGAAGGCGCCGCAAATACCATCGGCTGCGAGGCGACTGTGGTGGTCGAAGGAACG
14 15 16 17 18 19 20
TGATCCTTTGTCAGCTCAGCGGAGCCGAAGTGATCGCCGAGTTGGCTGAGCAGCGCCGAGG
21 22 23 24 25 26
GAGTTTAGAATCCGCTGCAGAAAACCTACCAGCTTCTTCGCGGACACAGCAGGTAATGTTCT
27 28 29
TAGGCTTCTGGAGTTAGTGAGTAGCCGGCCTGCAGGCCGGTAAATAACTCCGATCTAGCCC
30 31 32
CTTGGTTTATTTCTTTCGTTCGCGCCTTGAATGGAGGCGCCATGGCACAGTCTGGCAGAGGA
33 34 35 36
GGCCTGCCCTGGCGGGCGCTACTCAGGGCCCCTGCGTTGAGAATGCCGAGTGAGGTGCTGA
37 38 39 40
GGTTTGGAGAGAAAAAGGTGGCGTTTTCATCCCTTGGAATATCGGGCCTCTCCAGCTGAAG
41 42
CCTCCGGATCGACACAACACAACTCCCGGATCGACGCAACACAACTCCCCGCCAGCCCCTT
43 44 45 46 47 48
CCCTTCCAGCGGGCTGGACACAGGTGCGTGGGCCTCGCAGCCCGGCGCGCACGGTTGCGCC
49 50 51 52 53 54 55 56
TTTGGATACCTGCGACAGGACTCGGCCAGAACCGTGTCCCAGGGCAGAGAAAACCCTGGCA
57 58 59
ACCTTTAACGTTCTCCCTGACGCGGAGAATCCTGCGCCAACGGGGCCTTCCACTTCGGGAGA
60 61 62 63 64 65
CTCAATCGCCACCCGGTCCCCAACTTTCGTCTTCCAGAAGAAAAATCCATGAGGAGGAATG
66 67 68
GGGGGGTCCCCCGACCAGACCAGCAAGAAAGGGCCCAGTTAGGAGTGACCTCAGCGCCA
69 70
CGAGGGTTCCACTGCTTTGATGGCCACACGCTTTTGCAAACGGTTTGGGGGTGGAGAGACA
71 72 73
CCAGGTGATGGTAGGGAAGGGCGGTGGTGACTGGCACGGCTAAGACACTGCGGAGGGTTT
74 75 76
CGCTGTGGAGATGAGAAGGTGGAGGTTCTGGGCACTGTGGCTGCGAGTGTGAGCGATGCG
77 78 79 80
TTTGTGGGAATCAGAGGAGCGGATTTGGGAAAAGTGAATCCTTGAGATTCAGGGCCTTACT
81
TTCGTTAGGGGTGTGTGAAGCACACTGGGTGTCAGGAGAGGATGGGAATGGGATTCGAGAG
82 83
GCCTTTTTTTGGACTCCTCGAGAGAAAATGGAGAGAGTCTCGAACCCAGGAGATAGGAGG
84 85
CGTATTTTCCCCATGCACCCATGGATCACGCCCCCGCCCCCACATTCCCCCCGTAGGAGGCA
86 87 88 89
AGACCTGAGCGCTTACTCACGTGGCTCCCCCATCCGTTCCGCGTCCATCTCCAAGACTGCCT
90 91 92 93 94
CACAGGGACCCCCAGGAGGCTCCGAACCATCCAGCTTTCTGTCACCGCCGCCGCCACCAG
95 96 97 98
CGTTGTGAACCTCTGACCCTCGCGGCTCTGCGTCCATTCTCAGGTACTGAAAGTTTTCCGGG
99 100 101 102 103
CTCTTCCGCACCCGCGGATGTGGCGAAGCCGCGGGGCAGCTCCGCTCGCGCTCCAGTCGCA
104 105 106 107 108 109 110 111 112 113
GGATGTCCTTGACCGAGAAGGGGGTGGAGGTGACGGGGCTCAGCAGCATCCCGAAGGCGG
114 115 116 117
ATGGGGCGGGGCCGAGGAGGTCCGGGTGAGGAGCGGCACCCTGAACTTCCCGTCTTGTCGC
118 119 120 121 122 123

TGCAGGCCCCGCAGACAGACCCAAGCTCTGGGACAGACGCCCAGCGTCCCAGACAGCGCCT
124 125 126 127
TCCTCTGGGCCATGCTGGTAGGCCCGGGTCCAGGGCCGGGTGACGAGACCGTAGCCCCCCA
128 129 130 131
TTGGTTCTCGCAGAAACCACGTGCTAGCTCTGCACCTTCCTCCCCCAGCGCTTTCTTCCCGCG
132 133 134 135 136
CCCGTCGAATCCTCTCCAGTCCCCAGTCGCCTTCCTGGGAGGTTTGGTTCGTATTGACGGTT
137 138 139 140 141
CTCAAAGCAAAGAAAACGTGGAGAGACAGTGCTGCGCGCGGCTTGGATCTCCGTCAATACG
142 143 144 145 146 147
GTACTAGCGGGAGCTTCCGAGGTCAACCTAGGGTCCACGGGGACCCTCCTAGTGGACATG
148 149 150
CGGGGATTGGAGGTGGAGTCCTTGCTGTGCAACCCGATCCGGTCCATCCAGCCTGGTCTTAG
151 152 153
CTCAACTCTGCATTCTTATGGCGGAGGAAAGCTAAGATAGAGACAGCTGGGACCCGCGGAA
154 155 156
TTTGAGGCGCGCGCTGGGATCTTACGTGCGCAGAGGCCGGACCGCAGAGTAGAAGTGAGTC
157 158 159 160 161 162 163
TTGACCCACCAGGGCCTATATTGAGATGGAAACCTCTCTCTTTGAACAGAATCCAGGTCTG

CGGAGTCCCGACCCGCCACTTCCTAGGAGCAGCTTCTCAGCCCCAGGACCGAGGAATGGGT
164 165 166 167
GTGGGGGCTACATGTGGGGAGCCCTCGACCTCTTAAAGGGCTCCGCGAATGGTGGGGGAGA
168 169 170
CTGGAAGGCAATCTCTGCGGCCCGCGCAAAGTGGTCAGAGGCGGGAGTGGGGGTGGGGGG
171 172 173 174
GGGTGGGCTTCCGTCGCCCTTGGCGTCCCCACGTTCTTTTTCTTCCTCTCCTTTATCCTCCTTA
175 176 177 178
AAGCGGATTGCCTGGAGCGTGCCAGCTTGTGTGCGCGTATAAATGCGGCGCCCGCCGAAGG
179 180 181 182 183 184 185 186
GCTGGGAAGATTTGACTCGCATTAGATCTCCAGAAAAGGCCCTTGGTTACGGCGGGCACCG
187 188 189 190
AGTAAATGGCG
191

The bisulfite treated sequence of SEQ ID NO: 1 is shown in SEQ ID NO: 2 (Y represents C or U) as follows:

YGGATYGUTAUYGYGGUUUTGGGUTUAGTTUUTGGAGUYGGUUTYGGGTUYGGUUTYGGG
1 2 3 4 5 6 7 8
TUUUTUUTYGGYGGYGTUUAUTGUTGUYGUAGAAGAGAYGUTUUUAGGATTTGGTTTUUA
9 10 11 12 13
GTGGGAGAAGGAAGGYGUYGUAAATAUUATYGGUTGYGAGGYGAUTGTGGTGGTYGAAG
14 15 16 17 18 19
GAAYGTGATUUTTTGTUAGUTUAGYGGAGUYGAAGTGATYGUYGAGTTGGUTGAGUAGYG
20 21 22 23 24 25
UYGAGGGAGTTTAGAATUYGUTGUAGAAAAUUTAUUAGUTTUTTYGYGGAUAUAGUAGGT
26 27 28 29
AATGTTUTTAGGUTTUTGGAGTTAGTGAGTAGUYGGUUTGUAGGUYGGTAAATAAUTUYG
30 31 32
ATUTAGUUUUTTGGTTTATTTUTTTYGTTYGYGUUTTGAATGGAGGYGUUATGGUAUAGTU
33 34 35 36
TGGUAGAGGAGGUUTGUUUTGGYGGGYGUTAUTUAGGGUUUUTGYGTTGAGAATGUYGA
37 38 39 40
GTGAGGTGUTGAGGTTTGGAGAGAAAAAGGTGGYGTTTTUAUTUUTTGGAATATYGGGUUT
41 42
UTUUAGUTGAAGUUTUYGGATYGAUAUAAUAUAAUTUUYGGATYGAYGUAAUAUAAUTU
43 44 45 46 47
UUYGUUAGUUUUTTUUUTTUUAGYGGGUTGGAUAUAGGTGYGTGGGUUTYGUAGUUYGG
48 49 50 51 52

YGYGUAYGGTTGYGUUTTTGGATAUUTGYGAUAGGAUTYGGUUAGAAUYGTGTUUUAGGG
53 54 55 56 57 58 59
UAGAGAAAAUUUTGGUAAUUTTTAAYGTTUTUUUTGAYGYGGAGAATUUTGYGUUAAYGG
60 61 62 63 64
GGUUTTUUAUTTYGGGAGAUTUAATYGUUAUUYGGTUUUUAAUTTTYGTUTTUUAGAAGA
65 66 67 68
AAAATUUATGAGGAGGAATGGGGGGGTUUUUYGAUUAGAUUAGUAAGAAAGGGUUUAGT
69
TAGGAGTGAUUTUAGYGUUAYGAGGGTTUUAUTGUTTTGATGGUUAUAYGUTTTTGUAAA
70 71 72
YGGTTTGGGGGTGGAGAGAUAUUAGGTGATGGTAGGGAAGGGYGGTGGTGAUTGGUAYGG
73 74 75
UTAAGAUAUTGYGGAGGGTTTYGUTGTGGAGATGAGAAGGTGGAGGTTUTGGGUAUTGTG
76 77
GUTGYGAGTGTGAGYGATGYGTTTGTGGGAATUAGAGGAGYGGATTTGGGAAAAGTGAAT
78 79 80 81
UUTTGAGATTUAGGGUUTTAUTTTYGTTAGGGGTGTGTGAAGUAUAUTGGGTGTUAGGAGA
82
GGATGGGAATGGGATTYGAGAGGUUTTTTTTUGGAUTUUTYGAGAGAAAATGGAGAGAGT
83 84
UTYGAAUUUAGGAGATAGGAGGYGTATTTTUUUUATGUAUUUATGGATUAYGUUUUYGUU
85 86 87 88
UUUAUATTUUUUUYGTAGGAGGUAAGAUUTGAGYGUTTAUTUAYGTGGUTUUUUUATUYG
89 90 91 92
TTUYGYGTUUATUTUUAAGAUTGUUTUAUAGGGAUUUUUAGGAGGUTUYGAAUUATUUA
93 94 95
GUTTTUTGTUAUYGUYGUYGUUAUUAGYGTTGTGAAUUTUTGAUUUTYGYGGUTUTGYGT
96 97 98 99 100 101 102
UUATTUTUAGGTAUTGAAAGTTTTUYGGGUTUTTUYGUAUUYGYGGATGTGGYGAAGUYG
103 104 105 106 107 108
YGGGGUAGUTUYGUTYGYGUTUUAGTYGUAGGATGTUUTTGAUYGAGAAGGGGGTGGAG
109 110 111 112 113 114
GTGAYGGGGUTUAGUAGUATUUYGAAGGYGGATGGGGYGGGGUYGAGGAGGTUYGGGTG
115 116 117 118 119 120

AGGAGYGGUAUUUTGAAUTTUUYGTUTTGTYGUTGUAGGUUUYGUAGAUAGAUUUAAGU
121 122 123 124
TUTGGGAUAGAYGUUUAGYGTUUUAGAUAGYGUUTTUUTUTGGGUUATGUTGGTAGGUU
125 126 127
YGGGTUUAGGGUYGGGTGAYGAGAUYGTAGUUUUUUATTGGTTUTYGUAGAAAUUAYGT
128 129 130 131 132 133
GUTAGUTUTGUAUUTTUUTUUUUUAGYGUTTTUTTUUYGYGUUYGTYGAATUUTUTUUAGT
134 135 136 137 138
UUUUAGTYGUUTTUUTGGGAGGTTTGGTTYGTATTGAYGGTTUTUAAAGUAAAGAAAAYGT
139 140 141 142
GGAGAGAUAGTGUTGYGYGYGGUTTGGATUTUYGTUAATAYGGTAUTAGYGGGAGUTTU
143 144 145 146 147 148
YGAGGTUAAUUTAGGGTUUAYGGGGAUUUTUUTAGTGGAUATGYGGGGATTGGAGGTGGA
149 150 151
GTUUTTGUTGTGUAAUUYGATUYGGTUUATUUAGUUTGGTUTTAGUTUAAUTUTGUATTUT
152 153
TATGGYGGAGGAAAGUTAAGATAGAGAUAGUTGGGAUUYGYGGAATTTGAGGYGYGYGU
154 155 156 157 158 159
TGGGATUTTAYGTGYGUAGAGGUYGGAUYGUAGAGTAGAAGTGAGTUTTGAUUUAUUAGG
160 161 162 163
GUUTATATTGAGATGGAAAUUTUTUTUTTTGAAUAGAATUUAGGTUTGYGGAGTUUYGAU
164 165
UYGUUAUTTUUTAGGAGUAGUTTUTUAGUUUUAGGAUYGAGGAATGGGTGTGGGGGUTAU
166 167
ATGTGGGGAGUUUTYGAUUTUTTAAAGGGUTUYGYGAATGGTGGGGGAGAUTGGAAGGUA
168 169 170
ATUTUTGYGGUUYGYGUAAAGTGGTUAGAGGYGGGAGTGGGGGTGGGGGGGGGTGGGUTT
171 172 173 174
UYGTYGUUUTTGGYGTUUUUAYGTTUTTTTTUTTUUTUTUUTTTATUUTUUTTAAAGYGGAT
175 176 177 178 179
TGUUTGGAGYGTGUUAGUTTGTGTGYGYGTATAAATGYGGYGUUYGUYGAAGGGUTGGGA
180 181 182 183 184 185 186
AGATTTGAUTYGUATTAGATUTUUAGAAAAGGUUUTUGGTTAYGGYGGGUAUYGAGTAAA
187 188 189 190
TGGYG
191

The reverse complementary sequence of SEQ ID NO: 1 is shown in SEQ ID NO: 3 as follows:

CGCCATTTACTCGGTGCCCGCCGTAACCAAGGGCCTTTTCTGGAGATCTAATGCGAGTCAAATC
TTCCCAGCCCTTCGGCGGGCGCCGCATTTATACGCGCACACAAGCTGGCACGCTCCAGGCAATC
CGCTTTAAGGAGGATAAAGGAGAGGAAGAAAAAGAACGTGGGGACGCCAAGGGCGACGGAA
GCCCACCCCCCCCCACCCCCACTCCCGCCTCTGACCACTTTGCGCGGGCCGCAGAGATTGCCTT
CCAGTCTCCCCCACCATTCGCGGAGCCCTTTAAGAGGTCGAGGGCTCCCCACATGTAGCCCCCA
CACCCATTCCTCGGTCCTGGGGCTGAGAAGCTGCTCCTAGGAAGTGGCGGGTCGGGACTCCGC
AGACCTGGATTCTGTTCAAAGAGAGAGGTTTCCATCTCAATATAGGCCCTGGTGGGTCAAGACT
CACTTCTACTCTGCGGTCCGGCCTCTGCGCACGTAAGATCCCAGCGCGCGCCTCAAATTCCGCG
GGTCCCAGCTGTCTCTATCTTAGCTTTCCTCCGCCATAAGAATGCAGAGTTGAGCTAAGACCAG
GCTGGATGGACCGGATCGGGTTGCACAGCAAGGACTCCACCTCCAATCCCCGCATGTCCACTA
GGAGGGTCCCCGTGGACCCTAGGTTGACCTCGGAAGCTCCCGCTAGTACCGTATTGACGGAGA
TCCAAGCCGCGCGCAGCACTGTCTCTCCACGTTTTCTTTGCTTTGAGAACCGTCAATACGAACC
AAACCTCCCAGGAAGGCGACTGGGGACTGGAGAGGATTCGACGGGCGCGGGAAGAAAGCGCT
GGGGGAGGAAGGTGCAGAGCTAGCACGTGGTTTCTGCGAGAACCAATGGGGGGCTACGGTCTC
GTCACCCGGCCCTGGACCCGGGCCTACCAGCATGGCCCAGAGGAAGGCGCTGTCTGGGACGCT
GGGCGTCTGTCCCAGAGCTTGGGTCTGTCTGCGGGGCCTGCAGCGACAAGACGGGAAGTTCAG
GGTGCCGCTCCTCACCCGGACCTCCTCGGCCCCGCCCCATCCGCCTTCGGGATGCTGCTGAGCC
CCGTCACCTCCACCCCCTTCTCGGTCAAGGACATCCTGCGACTGGAGCGCGAGCGGAGCTGCCC
CGCGGCTTCGCCACATCCGCGGGTGCGGAAGAGCCCGGAAAACTTTCAGTACCTGAGAATGGA
CGCAGAGCCGCGAGGGTCAGAGGTTCACAACGCTGGTGGCGGCGGCGGTGACAGAAAGCTGG
ATGGTTCGGAGCCTCCTGGGGGTCCCTGTGAGGCAGTCTTGGAGATGGACGCGGAACGGATGG
GGGAGCCACGTGAGTAAGCGCTCAGGTCTTGCCTCCTACGGGGGGAATGTGGGGGCGGGGGCG
TGATCCATGGGTGCATGGGGAAAATACGCCTCCTATCTCCTGGGTTCGAGACTCTCTCCATTTT
CTCTCGAGGAGTCCAAAAAAAGGCCTCTCGAATCCCATTCCCATCCTCTCCTGACACCCAGTGT
GCTTCACACACCCCTAACGAAAGTAAGGCCCTGAATCTCAAGGATTCACTTTTCCCAAATCCGC
TCCTCTGATTCCCACAAACGCATCGCTCACACTCGCAGCCACAGTGCCCAGAACCTCCACCTTC
TCATCTCCACAGCGAAACCCTCCGCAGTGTCTTAGCCGTGCCAGTCACCACCGCCCTTCCCTAC
CATCACCTGGTGTCTCTCCACCCCCAAACCGTTTGCAAAAGCGTGTGGCCATCAAAGCAGTGGA
ACCCTCGTGGCGCTGAGGTCACTCCTAACTGGGCCCTTTCTTGCTGGTCTGGTCGGGGGACCCC
CCCATTCCTCCTCATGGATTTTTCTTCTGGAAGACGAAAGTTGGGGACCGGGTGGCGATTGAGT

CTCCCGAAGTGGAAGGCCCCGTTGGCGCAGGATTCTCCGCGTCAGGGAGAACGTTAAAGGTTG
CCAGGGTTTTCTCTGCCCTGGGACACGGTTCTGGCCGAGTCCTGTCGCAGGTATCCAAAGGCGC
AACCGTGCGCGCCGGGCTGCGAGGCCCACGCACCTGTGTCCAGCCCGCTGGAAGGGAAGGGGC
TGGCGGGGAGTTGTGTTGCGTCGATCCGGGAGTTGTGTTGTGTCGATCCGGAGGCTTCAGCTGG
AGAGGCCCGATATTCCAAGGGATGAAAACGCCACCTTTTTCTCTCCAAACCTCAGCACCTCACT
CGGCATTCTCAACGCAGGGGCCCTGAGTAGCGCCCGCCAGGGCAGGCCTCCTCTGCCAGACTG
TGCCATGGCGCCTCCATTCAAGGCGCGAACGAAAGAAATAAACCAAGGGGCTAGATCGGAGTT
ATTTACCGGCCTGCAGGCCGGCTACTCACTAACTCCAGAAGCCTAAGAACATTACCTGCTGTGT
CCGCGAAGAAGCTGGTAGGTTTTCTGCAGCGGATTCTAAACTCCCTCGGCGCTGCTCAGCCAAC
TCGGCGATCACTTCGGCTCCGCTGAGCTGACAAAGGATCACGTTCCTTCGACCACCACAGTCGC
CTCGCAGCCGATGGTATTTGCGGCGCCTTCCTTCTCCCACTGGAAACCAAATCCTGGGAGCGTC
TCTTCTGCGGCAGCAGTGGACGCCGCCGAGGAGGGACCCGAGGCCGGACCCGAGGCCGGCTCC
AGGAACTGAGCCCAAGGCCGCGGTAGCGATCCG

The bisulfite treated sequence of SEQ ID NO: 3 is shown in SEQ ID NO: 4 (Y represents C or U) as follows:

YGUUATTTAUTYGGTGUUYGUYGTAAUUAAGGGUUUTTTUTGGAGATUTAATGYGAGTUAAA
TUTTUUUAGUUUTTYGGYGGGYGUYGUATTTATAYGYGUAUAUAAGUTGGUAYGUTUUAGGU
AATUYGUTTTAAGGAGGATAAAGGAGAGGAAGAAAAAGAAYGTGGGGAYGUUAAGGGYGAY
GGAAGUUUAUUUUUUUUUAUUUUUAUTUUYGUUTUTGAUUAUTTTGYGYGGGUYGUAGAGA
TTGUUTTUUAGTUTUUUUUAUUATTYGYGGAGUUUTTTAAGAGGTYGAGGGUTUUUUAUATG
TAGUUUUUAUAUUUATTUUTYGGTUUTGGGGUTGAGAAGUTGUTUUTAGGAAGTGGYGGGTY
GGGAUTUYGUAGAUUTGGATTUTGTTUAAAGAGAGAGGTTTUUATUTUAATATAGGUUUTGG
TGGGTUAAGAUTUAUTTUTAUTUTGYGGTUYGGUUTUTGYGUAYGTAAGATUUUAGYGYGYG
UUTUAAATTUYGYGGGTUUUAGUTGTUTUTATUTTAGUTTTUUTUYGUUATAAGAATGUAGAG
TTGAGUTAAGAUUAGGUTGGATGGAUYGGATYGGGTTGUAUAGUAAGGAUTUUAUTUUUAAT
UUUYGUATGTUUAUTAGGAGGGTUUUYGTGGAUUUTAGGTTGAUUTYGGAAGUTUUYGUTAG
TAUYGTATTGAYGGAGATUUAAGUYGYGYGUAGUAUTGTUTUTUUAYGUTTTUTTTGUTTTGA
GAAUYGTUAATAYGAAUUAAAUUTUUUAGGAAGGYGAUTGGGGAUTGGAGAGGATTYGAYG
GGYGYGGGAAGAAAGYGUTGGGGGAGGAAGGTGUAGAGUTAGUAYGTGGTTTUTGYGAGAA
UUAATGGGGGGUTAYGGTUTYGTUAUUYGGUUUTGGAUUYGGGUUTAUUAGUATGGUUUAG
AGGAAGGYGUTGTUTGGGAYGUTGGGYGTUTGTUUUAGAGUTTGGGTUTGTUTGYGGGGUUT
GUAGYGAUAAGAYGGGAAGTTUAGGGTGUYGUTUUTUAUUYGGAUUTUUTYGGUUUYGUUU
UATUYGUUTTYGGGATGUTGUTGAGUUUYGTUAUUTUUAUUUUUTTUTYGGTUAAGGAUATU
UTGYGAUTGGAGYGYGAGYGGAGUTGUUUYGYGGUTTYGUUAUATUYGYGGGTGYGGAAGA
GUUYGGAAAAUTTTUAGTAUUTGAGAATGGAYGUAGAGUYGYGAGGGTUAGAGGTTUAUAA
YGUTGGTGGYGGYGGYGGTGAUAGAAAGUTGGATGGTTYGGAGUUTUUTGGGGGTUUUTGTG
AGGUAGTUTTGGAGATGGAYGYGGAAYGGATGGGGGAGUUAYGTGAGTAAGYGUTUAGGTUT
TGUUTUUTAYGGGGGGAATGTGGGGGYGGGGGYGTGATUUATGGGTGUATGGGGAAAATAYG
UUTUUTATUTUUTGGGTTYGAGAUTUTUTUUATTTTUTUTYGAGGAGTUUAAAAAAAGGUUTU
TYGAATUUUATTUUUATUUTUTUUTGAUAUUUAGTGTGUTTUAUAUAUUUTUAAYGAAAGTA
AGGUUUTGAATUTUAAGGATTUAUTTTTUUUAAATUYGUTUUTUTGATTUUUAUAAAYGUAT
YGUTUAUAUTYGUAGUUAUAGTGUUUAGAAUUTUUAUUTTUTUATUTUUAUAGYGAAAUUUT
UYGUAGTGTUTTAGUYGTGUUAGTUAUUAUYGUUUTTUUUTAUUATUAUUTGGTGTUTUTUU
AUUUUUAAAUYGTTTGUAAAAGYGTGTGGUUATUAAAGUAGTGGAAUUUTYGTGGYGUTGAG
GTUAUTUUTAAUTGGGUUUTTTUTTGUTGGTUTGGTYGGGGGAUUUUUUUATTUUTUUTUATG
GATTTTTUTTUTGGAAGAYGAAAGTTGGGGAUYGGGTGGYGATTGAGTUTUUYGAAGTGGAA
GGUUUYGTTGGYGUAGGATTUTUYGYGTUAGGGAGAAYGTTAAAGGTTGUUAGGGTTTTUTU
TGUUUTGGGAUAYGGTTUTGGUYGAGTUUTGTYGUAGGTATUUAAAGGYGUAAUYGTGYGYG
UYGGGUTGYGAGGUUUAYGUAUUTGTGTUUAGUUYGUTGGAAGGGAAGGGGUTGGYGGGGA
GTTGTGTTGYGTYGAUTYGGGAGTTGTGTTGTGTYGATUYGGAGGUTTUAGUTGGAGAGGUUY
GATATTUUAAGGGATGAAAAYGUUAUUUTTTTUTUTUUAAAUUTUAGUAUUTUAUTYGGUAT
TUTUAAYGUAGGGGUUUTGAGTAGYGUUYGUUAGGGUAGGUUTUUTUTGUUAGAUTGUUU
ATGGYGUUTUUATTUAAGGYGYGAAYGAAAGAAATAAAUUAAGGGGUTAGATYGGAGTTATT
TAUYGGUUTGUAGGUYGGUTAUTUAUTAAUTUUAGAAGUUTAAGAAUATTAUUTGUTGTGTU
YGYGAAGAAGUTGGTAGGUTTTUTGUAGYGGATTUTAAAUTUUUTYGGYGUTGUTUAGUUAA

UTYGGYGATUAUTTYGGUTUYGUTGAGUTGAUAAAGGATUAYGTTUUTTYGAUUAUUAUAGT
YGUUTYGUAGUYGATGGTATTTGYGGYGUUTTUUTTUTUUUAUTGGAAAUUAAATUUTGGGA
GYGTUTUTTUTGYGGUAGUAGTGGAYGUYGUYGAGGAGGGAUUYGAGGUYGGAUUYGAGGU
YGGUTUUAGGAAUTGAGUUUAAGGUYGYGGTAGYGATUYG

### Example 2. Difference in methylation of STAMP-EP8 CpG sites between tumor cells and non-tumor cells -- Bisulfite Sequencing PCR (BSP)

Bisulfite Sequencing PCR comprises the following steps:
1. Genomic DNA was extracted from lung cancer cell line A549 and normal lung cell line MRC5;
2. The extracted genomic DNA of lung cancer cell line A549 and normal lung cell line MRC5 were treated with bisulfite and used as templates for subsequent PCR amplification;
3. Primers (SEQ ID NO: 5-14; Table 1)were designed according to SEQ ID NO: 1 and used for amplification;
4. After PCR amplification, 2% agarose gel electrophoresis was used to detect the specificity of the PCR fragments. The target fragments were recovered by gel cutting and connected to T vector which was transformed into competent *Escherichia coli.* The bacteria were spread on plate, and clones were selected the next day and sequenced. More than ten clones were selected for each fragment for Sanger sequencing.

**Table 1**

| Primer Name | 5'-3' Primer Sequence | Detected CpG site No. |
|---|---|---|
| STAMP-EP8-Sanger-Primer-F | ATGAAGTTTATYGAAGGTGTTT (SEQ ID NO: 5) | CpG 001-040 of SEQ ID NO: 1 |
| STAMP-EP8-Sanger-Primer-R | TTTCTCTCCAAACCTCAACACCTC (SEQ ID NO: 6) | |
| STAMP-EP8-Sanger-Primer-F | GAGGTGTTGAGGTTTGGAGAGAAA (SEQ ID NO: 7) | CpG 041-077 of SEQ ID NO: 1 |
| STAMP-EP8-Sanger-Primer-R | AACCTCCACCTTCTCATCTCCACA (SEQ ID NO: 8) | |
| STAMP-EP8-Sanger-Primer-F | TGTGGAGATGAGAAGGTGGAGGTT (SEQ ID NO: 9) | CpG 078-114 of SEQ ID NO: 1 |
| STAMP-EP8-Sanger-Primer-R | ATACTACTAAACCCCRTCACCT (SEQ ID NO: 10) | |
| STAMP-EP8-Sanger-Primer-F | ATYGAGAAGGGGGTGGAGGTGA (SEQ ID NO: 11) | CpG 115-153 of SEQ ID NO: 1 |
| STAMP-EP8-Sanger-Primer-R | AACTAAAACCAAACTAAATAAAC (SEQ ID NO: 12) | |
| STAMP-EP8-Sanger-Primer-F | GTTTATTTAGTTTGGTTTTAGTT (SEQ ID NO: 13) | CpG 154-191 of SEQ ID NO: 1 |
| STAMP-EP8-Sanger-Primer-R | TAATAACCTTTATACCACACTAC (SEQ ID NO: 14) | |

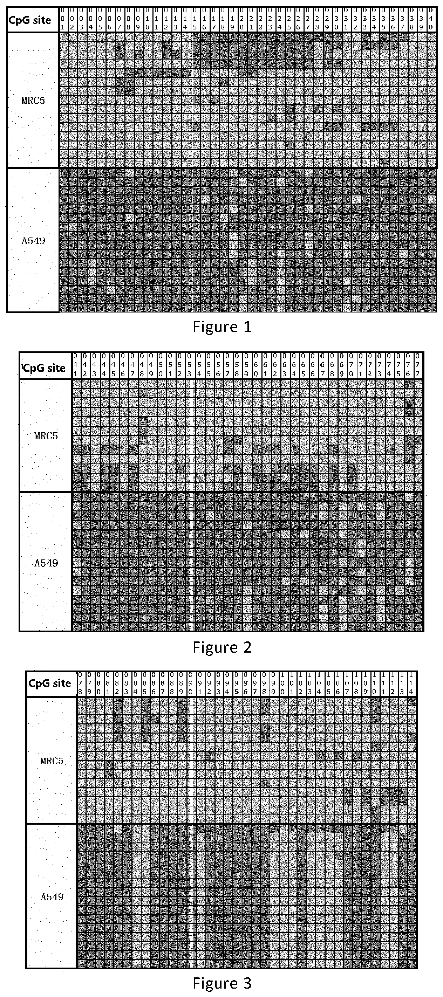

Figure 1 shows BSP assay for methylation levels of the methylation sites 001 to 040 in SEQ ID NO:1 region in lung cancer cells and normal lung cells, which indicates that the methylation level of STAMP-EP8 of lung cancer cells is significantly higher than that of normal lung cells.

Figure 2 shows BSP assay for methylation levels of the methylation sites 041 to 077 in SEQ ID NO:1 region in lung cancer cells and normal lung cells, which indicates that the methylation level of STAMP-EP8 of lung cancer cells is significantly higher than that of normal lung cells.

Figure 3 shows BSP assay for methylation levels of the methylation sites 078 to 114 in SEQ ID NO:1 region in lung cancer cells and normal lung cells, which indicates that the methylation level of STAMP-EP8 of lung cancer cells is significantly higher than that of normal lung cells.

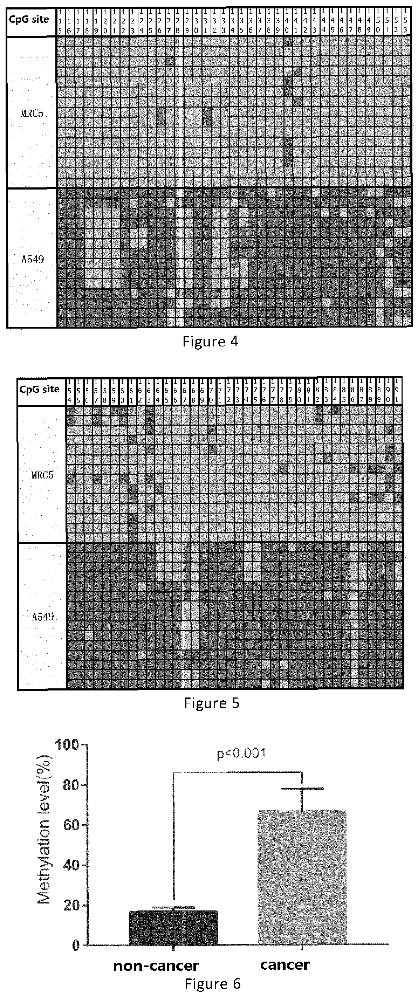

Figure 4 shows BSP assay for methylation levels of the methylation sites 115 to 153 in SEQ ID NO:1 region in lung cancer cells and normal lung cells, which indicates that the methylation level of STAMP-EP8 of lung cancer cells is significantly higher than that of normal lung cells.

Figure 5 shows BSP assay for methylation levels of the methylation sites 154 to 191 in SEQ ID NO:1 region in lung cancer cells and normal lung cells, which indicates that the methylation level of STAMP-EP8 of lung cancer cells is significantly higher than that of normal lung cells.

### Example 3: Difference in methylation of STAMP-EP8 CpG sites between tumor cells and non-tumor cells-pyrosequencing

Pyrosequencing comprises the following steps:
1. Clinical samples: paracancerous/non-cancer were used as the control group, and cancer tissue samples were used as the experimental group;
2. DNA extraction: DNA was extracted from the experimental group and the control group respectively. Phenol-chloroform extraction method was used in this experiment, which is not limited thereto;
3. Bisulfite treatment: the extracted DNA samples were treated with bisulfite and the procedures were strictly followed. EZ DNA Methylation-Gold Kit (ZYMO Research, Cat# D5006) was used in this experiment, which is not limited thereto;
4. Primer design: PCR primers and pyrosequencing primers were designed according to the characteristics of SEQ ID NO: 1 of STAMP-EP8 sequence. The methylation values of STAMP-EP8 were detected as the methylation values of CpG sites 021-024. The PCR primers sequences, pyrosequencing primers sequences, and the pyrosequencing detecting sequences and the detected sites are shown as SEQ ID NO: 15-18 (Table 2); PCR amplification and agarose gel electrophoresis: The bisulfite treated samples were used as templates for PCR amplification. The specificity of PCR amplification was identified by agarose gel electrophoresis of the amplified products;
5. PCR amplification and agarose gel electrophoresis: The bisulfite treated samples were used as templates for PCR amplification. The specificity of PCR amplification was identified by agarose gel electrophoresis of the amplified products;
6. Pyrosequencing: Pyro Mark Q96 ID pyrosequencing instrument (QIAGEN) was used for sequencing, and the procedures in instructions were strictly followed;
7. Calculation of STAMP-EP8 methylation value: pyrosequencing can detect the methylation value of each site in the target region, respectively, and the average methylation value of all sites were calculated as the STAMP-EP8 methylation value in the sample;
8. Results analysis: the methylation value of STAMP-EP8 was compared between the paracancerous/non-cancer control group and the cancer experimental group.

**Table 2**

| Primer Name | 5'-3' sequence | Detected CpG site No. |
|---|---|---|
| STAMP-EP8-Pyroseq-Primer-F | GAGGYGATTGTGGTGGTYGAAGGAA (SEQ ID NO: 15) | The primer pair detects CpG 021-024 of SEQ ID NO: 1 |
| STAMP-EP8-Pyroseq-Primer-R (with 5'-Biotin modification) | AAACTCCCTCRACRCTACTCAACCAACTC (SEQ ID NO:16) | |
| STAMP-EP8-Pyroseq-Primer-Seq | GAAGGAAYGTGATTTTTTGTTAGTTTAG (SEQ ID NO: 17) | |
| pyrosequencing detecting sequences | YGGAGTYGAAGTGATYGTYGAGTTGGTTGAGTAG YGTYGA (SEQ ID NO: 18) | |

### Example 4. Leukemia clinical sample assay - pyrosequencing

STAMP-EP8 methylation value between the control group of 8 non-leukemia bone marrow smear clinical samples and the experimental group of 8 leukemia bone marrow smear clinical samples was compared according to the pyrosequencing in Example 3.

The result in Figure 6 shows that, in clinical samples of leukemia, the methylation value of STAMP-EP8 in the experimental group was significantly higher than that of non-cancer tissues.

### Example 5. Breast cancer clinical sample assay - pyrosequencing

STAMP-EP8 methylation value between the control group of 5 cases of breast cancer paracancerous clinical samples and the experimental group of 5 cases of breast cancer clinical samples was compared according to the pyrosequencing in Example 3.

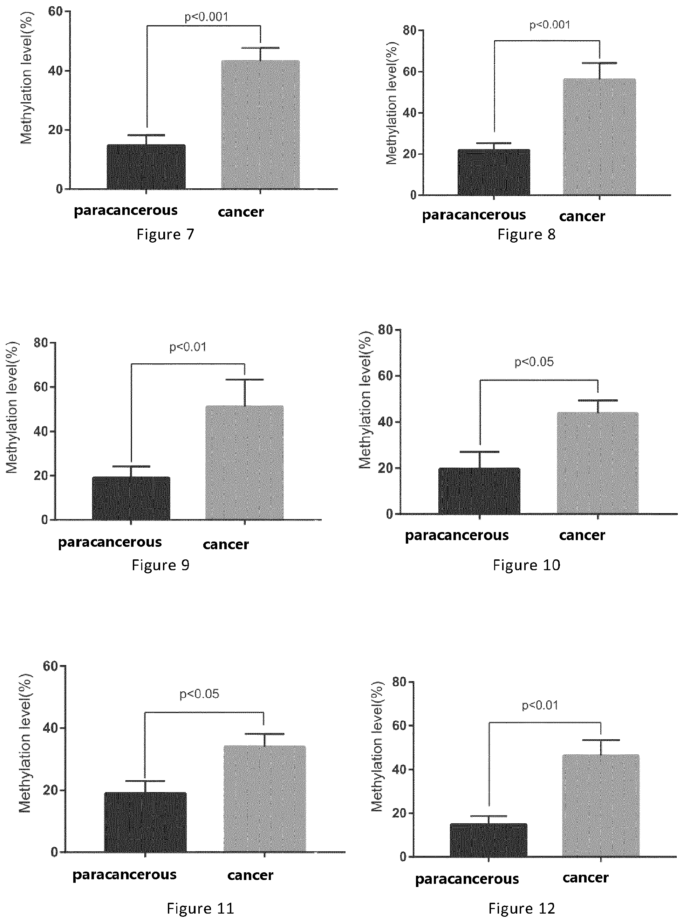

The result in Figure 7 shows that, in clinical samples of breast cancer, the methylation value of STAMP-EP8 in the experimental group was significantly higher than that of paracancerous tissues.

### Example 6. Colorectal cancer clinical sample assay - pyrosequencing

STAMP-EP8 methylation value was analyzed on the control group of 8 cases of colorectal cancer paracancerous clinical samples and the experimental group of 8 cases of colorectal cancer clinical samples according to the pyrosequencing in Example 3.

The result in Figure 8 shows that, in clinical samples of colorectal cancer, the methylation value of STAMP-EP8 in the experimental group was significantly higher than that of paracancerous tissues.

### Example 7. Esophageal cancer clinical sample assay - pyrosequencing

STAMP-EP8 methylation value was analyzed on the control group of 10 cases of esophageal cancer paracancerous clinical samples and the experimental group of 10 cases of esophageal cancer clinical samples according to the pyrosequencing in Example 3.

The result in Figure 9 shows that, in clinical samples of esophageal cancer, the methylation value of STAMP-EP8 in the experimental group was significantly higher than that of paracancerous tissues.

### Example 8. Liver cancer clinical sample assay - pyrosequencing

STAMP-EP8 methylation value was analyzed on the control group of 8 cases of liver cancer paracancerous clinical samples and the experimental group of 8 cases of liver cancer clinical samples according to the pyrosequencing in Example 3.

The result in Figure 10 shows that, in clinical samples of liver cancer, the methylation value of STAMP-EP8 in the experimental group was significantly higher than that of paracancerous tissues.

### Example 9. Lung cancer clinical sample assay - pyrosequencing

STAMP-EP8 methylation value was analyzed on the control group of 4 cases of lung cancer paracancerous clinical samples and the experimental group of 4 cases of lung cancer clinical samples according to the pyrosequencing in Example 3.

The result in Figure 11 shows that, in clinical samples of lung cancer, the methylation value of STAMP-EP8 in the experimental group was significantly higher than that of paracancerous tissues.

### Example 10. Pancreatic cancer clinical sample assay - pyrosequencing

STAMP-EP8 methylation value was analyzed on the control group of 4 cases of pancreatic cancer paracancerous clinical samples and the experimental group of 4 cases of pancreatic cancer clinical samples according to the pyrosequencing in Example 3.

The result in Figure 12 shows that, in clinical samples of pancreatic cancer, the methylation value of STAMP-EP8 in the experimental group was significantly higher than that of paracancerous tissues.

## Claims

1. A method for pan-cancer detection *in vitro,* comprising detecting the 5-methylation profile of a target sequence, wherein said target sequence is selected from:
(i) a polynucleotide, its nucleotide sequence is shown in SEQ ID No. 1,
(ii) a fragment of the polynucleotide of (i) consisting of or containing the bases at positions 204 to 223 or 1 to 478 of SEQ ID NO: 1; and/or
(iii) a nucleic acid complementary to the polynucleotide or fragment of (i) - (ii).

2. The method for pan-cancer detection *in vitro* according to claim 1, wherein said target sequence is a fragment of the polynucleotide of (i) consisting of bases at positions 204 to 223.

3. The method for pan-cancer detection *in vitro* according to claim 1 or 2, wherein said pan-cancer comprises at least a cancer selected from the group consisting of: hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma and thyroid cancer.

4. The method for pan-cancer detection *in vitro* according to any one of claims 1 - 3, comprising detecting the 5-methylation profile of a sample *in vitro,* comprising:
(i) providing the sample and extracting nucleic acid;
(ii) detecting the 5-methylation profile on a CpG site(s) of the target sequence(s) according to claim 1 or 2, preferably wherein said target sequence is a fragment of the polynucleotide of (i) consisting of bases at positions 204 to 223.

5. The method for pan-cancer detection *in vitro* according to any one of claims 1-4, wherein detecting the 5-methylation profile comprises pyrosequencing, bisulfite conversion sequencing, a method using a methylation chip, qPCR, digital PCR, second generation sequencing, third generation sequencing, whole genome methylation sequencing, DNA enrichment detection, simplified bisulfite sequencing technology, HPLC, MassArray or methylation specific PCR.

6. The method for pan-cancer detection *in vitro* according to claim 4, wherein, step (ii) comprises:
treating the product of (i) to convert unmodified cytosine into uracil; preferably, treating the nucleic acid of step (i) with bisulfite; and
analyzing the 5-methylation profile of the target sequence in the nucleic acid.

7. The method for pan-cancer detection *in vitro* according to claim 4 or 6, wherein, step (ii) comprises the use of
primers shown in SEQ ID NO: 15 and 16;
primers shown in SEQ ID NO: 5 and 6.

8. The method for pan-cancer detection *in vitro* according to claim 7, wherein, step (ii) comprises the use of primers shown in SEQ ID NO: 15 and 16.

9. A method of preparing a pan-cancer 5-methylation detection agent, comprising providing one or more polynucleotides comprising the target sequence(s) according to claims 1-2 and designing a detection agent for specifically detecting said target sequence(s), wherein the target sequence has at least the bases at positions 204 to 223 of SEQ ID NO: 1; wherein the detection agent comprises primers or probes.

10. A combination of pan-cancer 5-methylation detection agents, wherein the combination of agents specifically detects the 5-methylation on CpG site(s) of said target sequence(s) according to any one of claims 1-2, wherein said combination of detection agents are: the primers shown in SEQ ID NO: 15 and 16; or the primers shown in SEQ ID NO: 5 and 6.

11. The combination of pan-cancer 5-methylation detection agents according to claim 10 for use in pan-cancer detection, wherein said pan-cancer comprises at least a cancer selected from the group consisting of hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

12. Use of one or more polynucleotides comprising the sequence(s) according to any one of claims 1-2, and a combination of pan-cancer 5-methylation detection agents according to claim 10, in the manufacture of a pan-cancer detection kit, preferably wherein said pan-cancer comprises at least a cancer selected from the group consisting of hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

13. The use according to claim 12, wherein samples of the cancer comprise: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

14. A pan-cancer 5-methylation detection kit, comprising
container(s) and the combination of pan-cancer 5-methylation detection agents, according to claim 10 in the container(s).

## Patentansprüche

1. Verfahren zum In-vitro-Nachweis von Krebserkrankungen aller Art, das den Nachweis des 5-Methylierungsprofils einer Zielsequenz umfasst, wobei die Zielsequenz ausgewählt ist aus:
(i) einem Polynukleotid, dessen Nukleotidsequenz in SEQ ID NO: 1 dargestellt ist,
(ii) einem Fragment des Polynukleotids aus (i), das aus den Basen an den Positionen 204 bis 223 oder 1 bis 478 von SEQ ID NO: 1 besteht oder diese enthält; und/oder
(iii) einer Nukleinsäure in Ergänzung zu dem Polynukleotid oder dem Fragment aus (i) - (ii).

2. Verfahren zum In-vitro-Nachweis von Krebserkrankungen aller Art nach Anspruch 1, wobei die Zielsequenz ein Fragment des Polynukleotids (i) ist, das aus den Basen an den Positionen 204 bis 223 besteht.

3. Verfahren zum In-vitro-Nachweis von Krebs aller Art nach Anspruch 1 oder 2, wobei der Krebs mindestens eine Krebsart umfasst, die aus der Gruppe ausgewählt ist, die folgende Erkrankungen umfasst:
hämatologische Krebserkrankungen wie Leukämie, Lymphom, Multiples Myelom;
Tumore des gynäkologischen und reproduktiven Systems wie Brustkrebs, Eierstockkrebs, Gebärmutterhalskrebs, Vulvakrebs, Hodenkrebs, Prostatakrebs, Peniskrebs; Tumore des Verdauungssystems wie Speiseröhrenkrebs, Magenkrebs, Darmkrebs, Leberkrebs, Bauchspeicheldrüsenkrebs, Gallengangs- und Gallenblasenkrebs; Tumore der Atemwege wie Lungenkrebs, Brustfellkrebs;
Tumore des Nervensystems wie Gliom, Neuroblastom, Meningeom; Tumore im Kopf- und Halsbereich wie Mundhöhlenkrebs, Zungenkrebs, Kehlkopfkrebs und nasopharyngealer Krebs; Tumore des Harnsystems wie Nierenkrebs und Blasenkrebs; sowie Tumore der Haut und anderer Organsysteme wie Hautkrebs, Melanom, Osteosarkom, Liposarkom und Schilddrüsenkrebs.

4. Verfahren zum In-vitro-Nachweis von Krebserkrankungen aller Art nach einem der Ansprüche 1 bis 3, einschließlich des In-vitro-Nachweises des 5-Methylierungsprofils einer Probe, das Folgendes umfasst:
(i) Bereitstellung der Probe und Extrahieren von Nukleinsäure;
(ii) Nachweis des 5-Methylierungsprofils an einer oder mehreren CpG-Stelle(n) der Zielsequenz(en) nach Anspruch 1 oder 2, wobei die Zielsequenz vorzugsweise ein Fragment des Polynukleotids aus (i) ist, das aus den Basen an den Positionen 204 bis 223 besteht.

5. Verfahren zum In-vitro-Nachweis von Krebserkrankungen aller Art nach einem der Ansprüche 1 bis 4, wobei der Nachweis des 5-Methylierungsprofils die Pyrosequenzierung, die Bisulfit-Sequenzierung, ein Verfahren unter Verwendung eines Methylierungs-Chips, die qPCR, die digitale PCR, die Sequenzierung der zweiten Generation, die Sequenzierung der dritten Generation, die Gesamtgenom-Methylierungssequenzierung, den DNA-Anreicherungsnachweis, die vereinfachte Bisulfit-Sequenzierungstechnologie, die HPLC, das MassArray-System oder die methylierungsspezifische PCR beinhaltet.

6. Verfahren zum In-vitro-Nachweis von Krebserkrankungen aller Art nach Anspruch 4, wobei Schritt (ii) Folgendes umfasst:
Behandlung des Produkts aus (i), um unverändertes Cytosin in Uracil umzuwandeln; vorzugsweise Behandlung der Nukleinsäure aus Schritt (i) mit Bisulfit; und
Analyse des 5-Methylierungsprofils der Zielsequenz in der Nukleinsäure.

7. Verfahren zum In-vitro-Nachweis von Krebserkrankungen aller Art nach Anspruch 4 oder 6, wobei Schritt (ii) die Verwendung von
Primern nach SEQ ID NO: 15 und 16;
Primern nach SEQ ID NO: 5 und 6 umfasst.

8. Verfahren zum In-vitro-Nachweis von Krebserkrankungen aller Art nach Anspruch 7, wobei Schritt (ii) die Verwendung der in SEQ ID NO: 15 und 16 gezeigten Primer umfasst.

9. Verfahren zur Herstellung eines 5-Methylierungs-Nachweismittels für verschiedene Krebsarten, das die Bereitstellung eines oder mehrerer Polynukleotide mit der/den Zielsequenz(en) nach den Ansprüchen 1-2 und die Entwicklung eines Nachweismittels zum spezifischen Nachweis der Zielsequenz(en) umfasst, wobei die Zielsequenz mindestens die Basen an den Positionen 204 bis 223 von SEQ ID NO: 1 aufweist und das Nachweismittel Primer oder Sonden umfasst.

10. Kombination von Mitteln zum Nachweis der 5-Methylierung bei verschiedenen Krebsarten, wobei die Kombination von Mitteln die 5-Methylierung an der/den CpG-Stelle(n) der Zielsequenz(en) nach einem der Ansprüche 1 bis 2 spezifisch nachweist, wobei die Kombination von Nachweismitteln Folgendes umfasst: die in SEQ ID NO: 15 und 16 dargestellten Primer; oder die in SEQ ID NO: 5 und 6 dargestellten Primer.

11. Kombination von Mitteln zum Nachweis von 5-Methylierung bei verschiedenen Krebsarten nach Anspruch 10 zur Verwendung beim Nachweis verschiedener Krebsarten, wobei die verschiedenen Krebsarten mindestens eine Krebsart umfassen, die aus folgender Gruppe ausgewählt ist: hämatologischen Krebsarten wie Leukämie, Lymphom, multiplem Myelom; Tumoren des gynäkologischen und reproduktiven Systems wie Brustkrebs, Eierstockkrebs, Gebärmutterhalskrebs, Vulvakrebs, Hodenkrebs, Prostatakrebs, Peniskrebs; Tumoren des Verdauungssystems wie Speiseröhrenkrebs, Magenkrebs, Darmkrebs, Leberkrebs, Bauchspeicheldrüsenkrebs, Gallengangs- und Gallenblasenkrebs; Tumoren des Atmungssystems wie Lungenkrebs, Brustfellkrebs; Tumoren des Nervensystems wie Gliom, Neuroblastom, Meningeom; Tumoren im Kopf- und Halsbereich wie Mundhöhlenkrebs, Zungenkrebs, Kehlkopfkrebs, nasopharyngealer Krebs; Tumoren des Harnsystems wie Nierenkrebs, Blasenkrebs; Hauttumoren und Tumoren anderer Systeme wie Hautkrebs, Melanom, Osteosarkom, Liposarkom, Schilddrüsenkrebs.

12. Verwendung eines oder mehrerer Polynukleotide, die die Sequenz(en) nach einem der Ansprüche 1 bis 2 umfassen, sowie einer Kombination von Mitteln zum Nachweis von 5-Methylierungen bei verschiedenen Krebsarten nach Anspruch 10 bei der Herstellung eines Nachweiskits für verschiedene Krebsarten, wobei der Begriff "verschiedene Krebsarten" mindestens eine Krebsart umfasst, die vorzugsweise aus folgender Gruppe ausgewählt ist: hämatologischen Krebsarten wie Leukämie, Lymphom und multiplem Myelom; Tumoren des gynäkologischen und reproduktiven Systems wie Brustkrebs, Eierstockkrebs, Gebärmutterhalskrebs und Vulvakrebs, Hodenkrebs, Prostatakrebs, Peniskrebs; Tumoren des Verdauungssystems wie Speiseröhrenkrebs, Magenkrebs, Darmkrebs, Leberkrebs, Bauchspeicheldrüsenkrebs, Gallengangs- und Gallenblasenkrebs; Tumoren der Atemwege wie Lungenkrebs, Brustfellkrebs; Tumoren des Nervensystems wie Gliom, Neuroblastom, Meningeom; Tumoren im Kopf- und Halsbereich wie Mundhöhlenkrebs, Zungenkrebs, Kehlkopfkrebs, nasopharyngealer Krebs; Tumoren des Harnsystems wie Nierenkrebs, Blasenkrebs; Hauttumoren und Tumoren anderer Organsysteme wie Hautkrebs, Melanom, Osteosarkom, Liposarkom, Schilddrüsenkrebs.

13. Verwendung nach Anspruch 12, wobei die Krebsproben Folgendes umfassen: Gewebeproben, in Paraffin eingebettete Proben, Blutproben, Proben von Pleuraergüssen und Alveolarlavageflüssigkeit, Aszites und Lavageflüssigkeitsproben, Gallenproben, Stuhlproben, Urinproben, Speichelproben, Sputumproben, Liquorproben, Zellabstrichproben, Abstrich- oder Bürstenproben aus dem Gebärmutterhals sowie Gewebe- und Zellbiopsieproben.

14. Ein Kit zum Nachweis von 5-Methylierung bei verschiedenen Krebsarten, bstehend aus
einem oder mehreren Behälter(n) und der Kombination von Mitteln zum Nachweis von 5-Methylierung bei verschiedenen Krebsarten nach Anspruch 10 in dem/den Behälter(n).

## Revendications

1. Procédé conçu pour la détection pancancéreuse *in vitro* comprenant la détection du profil de la 5-méthylation d'une séquence cible, procédé dans lequel ladite séquence cible est sélectionnée à partir :
(i) d'un polynucléotide, sa séquence de nucléotide est montrée dans la référence SEQ ID NO : 1,
(ii) d'un fragment du polynucléotide de (i) se composant des bases, ou bien les contenant, au niveau des positions 204 à 223 ou 1 à 478 de la référence SEQ ID NO : 1 ; et/ou
(iii) d'un acide nucléique complémentaire du polynucléotide ou du fragment de (i) - (ii).

2. Procédé conçu pour la détection pancancéreuse *in vitro* selon la revendication 1, dans lequel ladite séquence cible est un fragment du polynucléotide de (i) se composant de bases au niveau des positions 204 à 223.

3. Procédé conçu pour la détection pancancéreuse *in vitro* selon la revendication 1 ou 2, dans lequel ledit pancancer comprend au moins un cancer sélectionné parmi le groupe se composant : de cancers hématologiques tels que la leucémie, le lymphome, le myélome multiple ; se composant de tumeurs des systèmes gynécologique et reproducteur telles qu'un cancer du sein, un cancer des ovaires, un cancer du col de l'utérus, un cancer de la vulve, un cancer des testicules, un cancer de la prostate, un cancer du pénis ; de tumeurs du système digestif telles qu'un cancer de l'œsophage, un cancer gastrique, un cancer colorectal, un cancer du foie, un cancer du pancréas, un cancer des voies biliaires et de la vésicule biliaire ; de tumeurs du système respiratoire telles qu'un cancer du poumon, un pleurome ; de tumeurs du système nerveux telles qu'un gliome, un neuroblastome, un méningiome ; de tumeurs de la tête et du cou telles qu'un cancer de la bouche, un cancer de la langue, un cancer du larynx, un cancer du nasopharynx ; de tumeurs du système urinaire telles qu'un cancer du rein, un cancer de la vessie ; de tumeurs de la peau et d'autres systèmes telles qu'un cancer de la peau, un mélanome, un ostéosarcome, un liposarcome et un cancer de la thyroïde.

4. Procédé conçu pour la détection pancancéreuse *in vitro* selon l'une quelconque des revendications 1 à 3, comprenant la détection du profil de la 5-méthylation d'un échantillon *in vitro,* ledit procédé comprenant :
(i) la fourniture de l'échantillon et l'extraction de l'acide nucléique ;
(ii) la détection du profil de la 5-méthylation sur un ou plusieurs site(s) CpG de la ou des séquence(s) cible(s) selon la revendication 1 ou 2, de préférence procédé dans lequel ladite séquence cible est un fragment du polynucléotide de (i) se composant de bases au niveau des positions 204 à 223.

5. Procédé conçu pour la détection pancancéreuse *in vitro* selon l'une quelconque des revendications 1 à 4, dans lequel la détection du profil de la 5-méthylation comprend le pyroséquençage, le séquençage par conversion au bisulfite, un procédé utilisant une puce de méthylation, une qPCR, une PCR numérique, le séquençage de deuxième génération, le séquençage de troisième génération, le séquençage de la méthylation du génome entier, la détection de l'enrichissement de l'ADN, la technologie de séquençage au bisulfite simplifiée, la HPLC, le système MassArray ou une PCR spécifique de la méthylation.

6. Procédé conçu pour la détection pancancéreuse *in vitro* selon la revendication 4, dans lequel l'étape (ii) comprend :
le traitement du produit de (i) afin de convertir la cytosine non modifiée en uracile ; comprend de préférence le traitement de l'acide nucléique de l'étape (i) avec du bisulfite ; et
l'analyse du profil de la 5-méthylation de la séquence cible dans l'acide nucléique.

7. Procédé conçu pour la détection pancancéreuse *in vitro* selon la revendication 4 ou 6, dans lequel l'étape (ii) comprend l'utilisation :
d'amorces montrées dans la référence SEQ ID NO : 15 et 16 ;
d'amorces montrées dans la référence SEQ ID NO : 5 et 6.

8. Procédé conçu pour la détection pancancéreuse *in vitro* selon la revendication 7, dans lequel l'étape (ii) comprend l'utilisation d'amorces montrées dans la référence SEQ ID NO : 15 et 16.

9. Procédé de préparation d'un agent de détection de la 5-méthylation pancancéreuse, ledit procédé comprenant la fourniture d'un ou de plusieurs polynucléotides comprenant la ou les séquence(s) cible(s) selon les revendications 1 et 2, et comprenant la conception d'un agent de détection afin de détecter spécifiquement ladite ou lesdites séquence(s) cible(s), procédé dans lequel la séquence cible a au moins les bases au niveau des positions 204 à 223 de la référence SEQ ID NO : 1 ; procédé dans lequel l'agent de détection comprend des amorces ou des sondes.

10. combinaison d'agents de détection de la 5-méthylation pancancéreuse, où la combinaison d'agents détecte spécifiquement la 5-méthylation sur un ou plusieurs site(s) CpG de ladite ou desdites séquence(s) selon une des revendications 1 et 2, où ladite combinaison fait que les agents de détection sont : les amorces montrées dans la référence SEQ ID NO : 15 et 16 ; ou bien les amorces montrées dans la référence SEQ ID NO : 5 et 6.

11. combinaison d'agents de détection de la 5-méthylation pancancéreuse selon la revendication 10 pour une utilisation dans une détection pancancéreuse, dans laquelle ledit pancancer comprend au moins un cancer sélectionné parmi le groupe se composant de cancers hématologiques tels que la leucémie, le lymphome, le myélome multiple ; se composant de tumeurs des systèmes gynécologique et reproducteur telles qu'un cancer du sein, un cancer des ovaires, un cancer du col de l'utérus, un cancer de la vulve, un cancer des testicules, un cancer de la prostate, un cancer du pénis ; de tumeurs du système digestif telles qu'un cancer de l'œsophage, un cancer gastrique, un cancer colorectal, un cancer du foie, un cancer du pancréas, un cancer des voies biliaires et de la vésicule biliaire ; de tumeurs du système respiratoire telles qu'un cancer du poumon, un pleurome ; de tumeurs du système nerveux telles qu'un gliome, un neuroblastome, un méningiome ; de tumeurs de la tête et du cou telles qu'un cancer de la bouche, un cancer de la langue, un cancer du larynx, un cancer du nasopharynx ; de tumeurs du système urinaire telles qu'un cancer du rein, un cancer de la vessie ; de tumeurs de la peau et d'autres systèmes telles qu'un cancer de la peau, un mélanome, un ostéosarcome, un liposarcome, un cancer de la thyroïde.

12. utilisation d'un ou de plusieurs polynucléotides comprenant la ou les séquence (s) selon l'une des revendications 1 et 2, et comprenant une combinaison d'agents de détection de la 5-méthylation pancancéreuse selon la revendication 10, ladite utilisation étant prévue pour la fabrication d'un kit de détection pancancéreuse, de préférence où ledit pancancer comprend au moins un cancer sélectionné parmi le groupe se composant de cancers hématologiques tels que la leucémie, le lymphome, le myélome multiple ; se composant de tumeurs des systèmes gynécologique et reproducteur telles qu'un cancer du sein, un cancer des ovaires, un cancer du col de l'utérus, un cancer de la vulve, un cancer des testicules, un cancer de la prostate, un cancer du pénis ; de tumeurs du système digestif telles qu'un cancer de l'œsophage, un cancer gastrique, un cancer colorectal, un cancer du foie, un cancer du pancréas, un cancer des voies biliaires et de la vésicule biliaire ; de tumeurs du système respiratoire telles qu'un cancer du poumon, un pleurome ; de tumeurs du système nerveux telles qu'un gliome, un neuroblastome, un méningiome ; de tumeurs de la tête et du cou telles qu'un cancer de la bouche, un cancer de la langue, un cancer du larynx, un cancer du nasopharynx ; de tumeurs du système urinaire telles qu'un cancer du rein, un cancer de la vessie ; de tumeurs de la peau et d'autres systèmes telles qu'un cancer de la peau, un mélanome, un ostéosarcome, un liposarcome, un cancer de la thyroïde.

13. Utilisation selon la revendication 12, utilisation dans laquelle des échantillons du cancer comprennent : des échantillons de tissus, des échantillons inclus en paraffine, des échantillons de sang, des échantillons d'épanchement pleural et des échantillons de liquide de lavage alvéolaire, des échantillons d'ascite et de liquide de lavage, des échantillons de bile, des échantillons de selles, des échantillons d'urine, des échantillons de salive, des échantillons d'expectorations, des échantillons de liquide cérébrospinal, des échantillons de frottis cellulaires, des prélèvements par raclage ou brossage du col de l'utérus, des échantillons de biopsie tissulaire et cellulaire.

14. Kit de détection de la 5-méthylation pancancéreuse, ledit kit comprenant :
un ou plusieurs contenant(s) et la combinaison d'agents de détection de la 5-méthylation pancancéreuse selon la revendication 10, lesdits agents de détection étant placés dans le ou les contenant(s).
